# NEW EUROPEAN PATENT SPECIFICATION

(11) **EP 0 313 343 B2**
(45) Date of publication and mention of the opposition decision: **23.07.2003**
(45) Mention of the grant of the patent: 19.04.1995
(21) Application number: 88309824.6
(22) Date of filing: 19.10.1988
(51) Int. Cl.: C07K 1/18, C12N 15/27, B01J 39/06

(54) **Method of purifying protein**
Verfahren zur Proteinreinigung
Procédé de purification de protéines

(30) Priority: 23.10.1987 US 111886
(43) Date of publication of application: 26.04.1989
(73) Proprietor: SCHERING CORPORATION, Kenilworth New Jersey 07033 (US)
(72) Inventor: Naveh, David, Millburn New Jersey 07041 (US); Tang, John Chu-Tay, Livingston New Jersey 07039 (US)
(74) Representative: Ritter, Stephen David

(56) References cited:
- EP-A- 0 176 299
- EP-A- 0 254 399
- US-A- 4 658 018
- BIOCHEM. JOURNAL, vol. 240, pages 1-12, GB; F.A.O. MARSTON: "The purificationof eukaryotic polypeptides synthesized in Escherichia coli"
- LE TECHNOSCOPE DE BIOFUTUR, no. 12, July/August 1987, pages 4-6, Supplement,Paris, FR; "La chromatographie préparative basse pression"
- IMMUNOLOGICAL REV., vol. 63, 1982, pages 166-209; S. GILLIS et al.: "Molecularcharacterization of interleukin 2"
- BIOCHEMISTRY, second edition, 1981, page 19, W.H. Freeman and Co., New York, US
- Science 238 (1987) 319-323
- Pharmacia FPLC ionexchange and chromatofocusing, 1985, pp 63,64,88

## Description

### BACKGROUND

Ion-exchange chromatography is a conventional chromatographic technique, inherently mild, low cost, large capacity and readily scalable. However, to date it has not been a useful technique for separating closely related impurities from proteins, especially rDNA proteins.

An example of such an rDNA protein is human recombinant granulocyte macrophage colony stimulating factor (GM-CSF). Complementary DNAs (cDNAs) for GM-CSF, factors which support growth and development of granulocytes and macrophages in the blood, have recently been cloned and sequenced by a number of laboratories. Moreover, non-recombinant GM-CSF has been purified from culture supernatants of the Mo cell line (described in U.S. Patent 4,438,032), and the first sixteen amino acids from the N-terminus have been sequenced, Gasson et al., Science, Vol. 226, pgs. 1339-1342 (1984). Among the human GM-CSFs, nucleotide sequence and amino acid sequence heterogeneity have been observed. For example, at the amino acid level both threonine and isoleucine have been observed at position 100 with respect to the N-terminal alanine, suggesting that several allelic forms, or polymorphs, of GM-CSF may exist within human populations.

A variety of methods are now available for de novo preparation and cloning of cDNAs, such as cDNAs for GM-CSF, and for the construction of cDNA libraries. By way of example, total mRNA is extracted from cells (e.g., a nontransformed human T cell source) producing polypeptides exhibiting the desired activity. The double-stranded cDNAs can be constructed from this total mRNA by using primer-initiated reverse transcription to make first the complement of each mRNA sequence, and then by priming for second strand synthesis. Subsequently, the cDNAs can be cloned by joining them to suitable plasmid or bacteriophage vectors through complementary homopolymeric tails or cohesive ends created with linker segments containing appropriate restriction sites and then transtorming a suitable host. A wide range of expression systems (i.e., host-expression vector combinations) can be used to produce the proteins purified by the process of this invention. Possible types of host cells include, but are not limited to, bacterial, yeast, insect, mammalian and the like.

Various methods have been disclosed for extracting the GM-CSF from the host cells and subsequently purifying it, but GM-CSF is not always adequately purified in good yield and with retention of biological activity.

### SUMMARY OF THE INVENTION

The present invention is directed at a method for separating GM-CSF from Δ4 GM-CSF which comprises contacting a protein mixture comprising GM-CSF and Δ4 GM-CSF with a strong ion exchange resin, characterised in that the protein mixture is adjusted to a selected pH value at which only one of the GM-CSF and Δ4 GM-CSF is bound by the resin and the other is not, wherein said selected pH value lies between and is only fractional pH units away from the determined isoelectric points of the GM-CSF and Δ4 GM-CSF, whereby at said selected pH value the GM-CSF and Δ4 GM-CSF possess very small electrical charges, are oppositely charged, and only one of the GM-CSF and Δ4 GM-CSF binds to the ion exchange resin, and recovering GM-CSF.

The pH of the crude protein mixture is adjusted to a pH within the range of isoelectric points of the protein fractions to be separated such that there is an amplified net charge difference between the fractions.

The method of the present invention includes the step of determining the isoelectric points of the pure protein to be recovered and the impurity to be removed and determining the pH within the range of isoelectric points such that the pure protein and the impurity are oppositely charged and an amplified difference between said charges exists. The isoelectric points of the protein fractions preferably are determined by computer simulation or by using isoelectric focusing gels.

In a particularly preferred method, GM-CSF is purified by sequentially contacting the protein with:
A. an anion exchange resin such as a quarternary amine resin preferably attached to a cross-linked dextran, cellulose, agarose or acrylic support;
B. a cation exchange resin preferably having a sulfonate functionality attached to a cross-linked dextran, cellulose, agarose or acrylic support and
C. a gel filtration means preferably having a fractionation range of about 5,000 to about 100,000 daltons for proteins.

### DETAILED DESCRIPTION

The present invention relates to a high resolution ion-exchange chromatographic separation techique for purifying GM-CSF . In this technique, chromatography is carried out near the isoelectric points of the pure protein sought to be recovered and the impurity sought to be removed, preferably at a pH where an amplified (e.g. the maximum) difference between charges on the pure protein and impurity exists, and where the molecules are oppositely charged (polarized). This amplified net charge difference, which occurs only near the isoelectric point, can be used to select appropriate ion exchange chromatography conditions, resulting in selective binding to ion exchange resin in the near absence of charges. For convenience, this technique is hereafter referred to as Delta Isoelectric Point (DIP) chromatography.

rDNA impurities closely related to a desired rDNA protein are difficult to remove from crude protein by gel filtration chromatography, ion exchange chromatography, hydrophobic interaction chromatography, reversed-phase high performance liquid chromatography, metal chelating affinity chromatography as well as other conventional types of chromatography. These closely related impurities usually are proteins with a molecular weight and charge distribution close to the protein product of interest, usually missing one (or more) charged amino acids. Examples are impurities that are proteolytic degradation products formed by nicking a peptide terminus that is accessible to proteolysis as well as to chromatographic interaction. Such impurities are considered inseparable by conventional ion exchange chromatography.

The DIP chromatography technique differs from conventional ion exchange chromatography in a number of ways. DIP is performed at a pH where proteins to be separated possess very small net electrical charges. This is achieved by loading the ion exchange resin at a pH that is only fractional pH units away from the isoelectric point (pl) of the desired protein and its closely related impurities. This is in contrast to conventional ion exchange chromatography, which is performed at pH's where proteins of interest possess strong electrical charges, and wherein loading is carried out about 1-2 whole pH units away from the pl, an order of magnitude greater than in DIP chromatography.

The pH at which to perform DIP is predicted by computer simulations of charge density vs. pH for the protein and related impurities, or by analytical techniques such as the use of isoelectric focusing gels. In conventional ion exchange chromatography, loading pH is determined by gross net charge on the desired protein, and loading conditions are optimized empirically, if at all. Tedious trial and error experimentation is needed to determine elution conditions.

Unlike conventional ion exchange chromatography wherein no attempt is made to polarize the impurities relative to the desired protein, in DIP chromatography the loading pH is selected so that it lies between the pl of the closely related impurity and that of the desired protein, and therefore, the desired protein attains an opposite electrical charge to the polarized impurity.

For DIP chromatography, a high ionic strength environment is imposed to reduce protein-protein interactions during loading, and a strong ion exchanger is used. In conventional ion exchange technology, to obtain maximum resolution, weak ion exchange resins with lower ionic strength loading conditions are preferred. The low protein-protein interaction during loading in DIP chromatography results in highly selective binding and extremely fine resolution during the loading phase, and additional separation is attained during normal gradient elution. In conventional ion exchange chromatography, non-selective binding occurs during loading and therefore only group separation is achieved during loading; most of the separation is obtained during the elution phase, under strong protein-protein interactions resulting from high locally bound protein densities on the solid matrix.

Computer simulation of charge density vs. pH can be conducted using commercially available software and an appropriate computer (e.g. mainframe, microcomputer or personal computer). For example, a VAX computer (Digital Corp.) may be used with a software package such as "Polypeptide Analysis System" by Intellegentics, Inc., copyright 1981, 1982, 1983, 1984, 1985 and 1986. Using this software the primary amino acid sequence is the input and the charge density distribution can be obtained at different pH's. Alternatively, pl may be determined by using isoelectric focusing (IEF) gel electrophoresis according to procedures well known to those skilled in the art.

This patent relates to the application of DIP chromatography is the following procedure for the purification of human recombinant GM-CSF. A particularly difficult aspect of the purification of GM-CSF is the removal of a GM-CSF degradation product missing four N-terminal amino acids and known as Delta 4 (Δ4). GM-CSF is usually purified by a combination of anion exchange on a quaternary aminoethyl column, gel filtration and reversed phase chromatography, which procedure is not effective in removing the Δ4 impurity.

DIP chromatography, however, has been successful in removing the Δ4 impurity. First, a computer simulation of the intact GM-CSF protein was compared to the computer simulation of the Δ4 impurity, and it was determined that the pl of GM-CSF is 5.24 and the pl of the Δ4 impurity is 4.98. Next, a working pH within this range had to be determined at which (a) GM-CSF attained an opposite charge to the Δ4 impurity, and (b) the relative magnitude of the difference between the charges is sufficiently amplified to enable separation. A minute charge difference of 1 charge/molecule (1 ch/mol) between the intact GM-CSF and the Δ4 impurity exists over a broad pH range (i.e., at pH 0.5, the respective charges are +16 and +15 and at a pH 11.5, the charges are -11.1 and -12). However, as predicted by the computer model, close to pH 5 the charge difference between intact GM-CSF and the Δ4 impurity is amplified (i.e. the relative charge difference is increased) and the molecules are polarized: at pH 5, GM-CSF has +0.9 ch/mol, while the Δ4 impurity possesses only -0.1 ch/mol. Hence, the two necessary conditions for DIP are satisfied: charge polarization and amplification.

To further enhance the separation of intact GM-CSF from remaining low pl impurities of the E. coli host cells and from proteolytic destablizing factors, high ionic strength was employed during loading. This diminishes electrostatic interactions among the molecules. Under these high ionic strength conditions, a strong cation exchanger at pH 5, e.g. a column such as a sulfonate functionality attached to a cross-linked dextran, cellulose, agarose or acrylic support (e.g., S-Sepharose, manufactured by Pharmacia, Inc., Piscataway, NJ) is therefore preferred.

The use of such a cation exchange resin column was found to accomplish a one-step removal of the undesirable Δ4 impurity, various low pl impurities, a 20K E. coli impurity not previously removed by other procedures, and a proteolytic factor, thereby stabilizing the final product. Performing the cation exchange chromatography raises the purity of the GM-CSF from about 50% to about 90%, with yields of 70-80%. When followed by a gel filtration step, the purity of the GM-CSF is raised to about 99%.

Following is a general description of a procedure for the isolation and purification of GM-CSF. The anion exchange chromatography, preferably on a quaternary aminoethyl column, is a conventional step in the purification of GM-CSF and is performed to remove high pl impurities from the supernatant after the host cells are killed. The gel filtration step, also conventional, is performed to remove high and low molecular weight impurities. While the following describes a process in which the purified protein fraction is bound to the strong ion exchange resin, it is also contemplated that in certain embodiments of the invention the impure protein fraction may be bound to the strong ion exchange resin. Similarly, while the description and example are directed at the continuous passing of the crude protein through an ion exchange resin column, other contacting methods, such as batch contacting, also are contemplated.

General comments: Operations are performed at 2-15°C unless otherwise indicated. Protein concentration is determined at each stage by a Coomassie Blue binding assay. if the expected degree of purification is not achieved in any chromatographic procedure, eluted fractions may be re-chromatographed on the same column, or, alternatively, recycled through a previous step or a previous series of steps. This reprocessing can be done on eluted side fractions from a batch or a pool of batches. At any step, concentration may be performed by an ammonium sulfate preciptation, isoelectric point precipitation and/or ultrafiltration. Buffer solutions are made with deionized water (DI), reverse osmosis. water (RO), or water for injection (WFI).

### STAGE I: CHROMATOGRAPHY ON QUATERNARY AMINE COLUMN

The pH of the crude extract of GM-CSF is adjusted to 5-7.5 with buffer such as 1M Bis-tris (bis[2-hydroxyethyl]imino-tris-[hydroxymethyl]-methane) and/or 4N HCl. The solution is then clarified by centrifugation and/or filtration. The conductivity is adjusted to below 10 millisiemens/centimeter (mS/cm) by dilution with water or addition of a salt solution such as 4N NaCI. The batch is applied to a quaternary amine column (i.e., a quaternary amine functionality attached to a cross-linked dextran, cellulose, agarose or acrylic support, such as Q-Sepharose, manufactured by Pharmacia, Inc.) at a loading of not greater than 50 grams of protein per liter of gel. Elution is performed with a gradient in the range of 0-0.4M NaCl or other appropriate salt in a buffer such as 20mM Bis-tris. Appropriate fractions are combined for further processing.

### STAGE II: CHROMATOGRAPHY ON SULFONATE COLUMN

The combined fractions are adjusted to pH 5, which is within the range of isoelectric points of the protein fractions as determined by DIP chromatography, with an acid such as 1M acetic acid or 4N HCI or a base such as 6N NaOH. The conductivity is adjusted to 13 mS/cm with a buffer such as 0.01M acetic acid adjusted to pH 5 with NaOH. The solution is filtered through a 0.2 micron filter and charged onto a sulfonate column (i.e., a sulfonate functionality attached to a cross-linked dextran, cellulose, agarose or acrylic support such as S-Sepharose) at a loading of not greater than 20 grams of protein per liter of column material. Elution is performed with a solution of a salt such as NaCl at a concentration gradient up to 0.5M in a buffer such as 20mM acetic acid, 0.13M NaCI, pH 5.0 buffer. Appropriate fractions are combined for further processing. This chromatography step is typically repeated.

### STAGE. III: AMMONIUM SULFATE PRECIPITATION

Ammonium sulfate is added to the combined S-Sepharose fractions to a final concentration of 50% to 60% saturation. The precipitate is collected by centrifugation. The precipitate may be stored under refrigeration.

### STAGE IV: GEL FILTRATION CHROMATOGRAPHY

The ammonium sulfate precipitate is dissolved in a buffer such as 10mM sodium phosphate, 50mM citric acid, pH 6 buffer containing up to 0.35M of a salt such as sodium chloride. The solution is centrifuged and filtered through a 0.2 micron range filter prior to loading on to a gel filtration column having a fractionation range of from about 5,000 to about 100,000 daltons for proteins, e.g., Sephacryl S-200 HR (manufactured by Pharmacia, Inc.), pre-equllibrated with the same buffer. The loading is not greater than 3.5 grams of protein per liter of gel. The column is eluted with the same buffer and appropriate fractions are combined. The fractions are dialyzed against a buffer such as an 10mM phosphate, 2mM citrate, pH 7.2 buffer. Alternatively, the entire Stage IV can be performed in this latter buffer.

### STAGE V: PURIFIED BULK GM-CSF

The combined fractions are filtered through a 0.2 micron or smaller pore size filter and stored at -20°C or below.

Typical IEF gel electrophoresis procedures are disclosed in the following references:
1. ELECTROPHORETIC TECHNIQUES, Academic Press, (1983) Ed: G.F. Simpson & M. Whittaker "Recent Developments in Isoelectric-focusing" J.S. Fawcett, p. 57
2. ISOELECTRIC-FOCUSING: Theory, Methodology and Application P.G. Righetti, Elsevier Biomedical Press, (1983) p. 14B
3. APPLICATION OF SEPARATOR ISOELEC-TRIC-FOCUSiNG WITHIN pH RANGE 4-6, P. Gill, Electrophoresis 6:282 (1985)
4. RAPID STAINING OF PROTEINS IN ULTRA THIN ISOELECTRIC FOCUSING IN POLYACRYLAMIDE GEL, M.D. Frey, Electrophoresis 3:27-32 (1982)
5. ULTRA THIN LAYER ISOELECTRIC-FOCUSING OF ENZYMES IN LIVER SAMPLES OF WAGTAILS, M. Germeiner, Electrophoresis 3:146 (1982)

Below is presented an example illustrating procedures for purifying GM-CSF based on the DIP technique.

### EXAMPLE 1

### PURIFICATION OF GM-CSF

### Step 1: Quaternary Amine Column Chromatography

The pH of 180L of crude GM-CSF extract was adjusted to pH 6.0 with 3.6L 1M Bis-tris buffer (pH 6.0) and with 2.0L of 4N HCI. The solution was clarified by centrifugation using a Sharples centrifuge at a feed rate of 0.75L/min. The supernatant was diluted approximately 1.55 fold with cold deionized water to reach a final conductivity of 5.5 mS/cm. A 12L column of Q-Sepharose was equilibrated with 10 column volumes of 20mM Bis-tris buffer at pH 6.0, and 43.6L of extract was applied to the column at a loading of 20 mg protein per ml of resin. The column (diameter 25 cm) was washed at a flow rate of 250 ml/min. with 120L of equilibration buffer. A linear gradient was established between 78L of 20mM Bis-tris buffer containing 0.03 NaCI at pH 6.0 and 78L of 20mM Bis-tris buffer at pH 6.0 containing 0.32M NaCI. Fractions (1.2L each) were combined based on gel electrophoresis (SDS-PAGE) and the pooled protein (4.9L) was chromatographed in Step 2.

### Step 2: Sulfonate-Column Chromatography

The combined fractions (4.9L) from Step 1 were adjusted to pH 5 with 49 ml 1M acetic acid (pH 5.0) and the conductivity was adjusted to 15 mS/cm with 2L 0.01 M acetic acid adjusted to pH 5 with NaOH. The solution was filtered through a 0.2 micron filter and then charged to a 0.8L S-Sepharose column previously equilibrated with 20mM acetic acid containing 0.13M NaCI at pH 5 at a loading rate of 4.7 mg/ml column material. The column was washed with 2.4L equilibrating buffer, then eluted with a linear gradient established between 5.6L 20mM acetic acid at pH 5 containing 0.13M NaCl and 5.6L 20mM acetic acid at pH 5 containing 0.5M NaCl. The fractions were combined based on gel electrophoresis. This chromatography step was repeated.

### Step 3: Ammonium Sulfate Precipitation

Ammonium sulfate was added to the combined fractions from Step 2 (240L), giving a concentration of 351 g/L (55% saturation). The solution was held at 4°C without mixing for 2 hours, then was centrifuged at 4,500 rpm for 30 min at 4°C to obtain the precipitate.

### Step 4: Gel Filtration Chromatography

The ammonium sulfate pellet was dissolved in 36 ml of 18mM sodium phosphate, 2mM citric acid, pH 7.2 buffer. The solution was centrifuged at 4,500 rpm for 30 min. and the supernatant filtered through a 0.2 micron filter. The filtrate was loaded onto a 1.8L Sephacryl S-200HR column previously equilibrated with phosphate-citrate pH 7.2 buffer at a loading rate of 0.21 mg/ml gel. The column was eluted with 1.9L of the same pH 7.2 buffer. Fractions were combined based on protein assay.

### Step 5: Filtration

The combined fractions from Step 4 were filtered through a 0.2 micron fitter and stored at -20°C.

## Claims

1. A method for separating GM-CSF from Δ4 GM-CSF which comprises contacting a protein mixture comprising GM-CSF and Δ4 GM-CSF with a strorig ion exchange resin, **characterised in that** the protein mixture is adjusted to a selected pH value at which only one of the GM-CSF and Δ4 GM-CSF is bound by the resin and the other is not, wherein said selected pH value lies between and is only fractional pH units away from the determined isoelectric points of the GM-CSF and Δ4 GM-CSF, whereby at said selected pH value the GM-CSF and Δ4 GM-CSF possess very small electrical charges, are oppositely charged, and only one of the GM-CSF and Δ4 GM-CSF binds to the ion exchange resin, and recovering GM-CSF.

2. The method of Claims 1 in which the GM-CSF is bound to an anion exchange resin, eluted and then subjected to gel filtration column chromatography.

3. The method of any preceding claim in which the pH of the crude protein mixture is adjusted to pH 5.

## Patentansprüche

1. Verfahren zum Abtrennen von GM-CSF und Δ4GM-CSF, Schritte umfassend, bei denen man eine Proteinmischung, die GM-CSF und Δ4GM-CSF umfaßt, mit einem starken Ionenaustauscherharz umsetzt, **dadurch gekennzeichnet, daß** die Proteinmischung auf einen ausgewählten pH-Wert eingestellt wird, bei dem entweder nur GM-CSF oder Δ4GM-CSF an das Harz bindet und das jeweils andere nicht, wobei der ausgewählte pH-Wert zwischen den ermittelten isoelektrischen Punkten des GM-CSF und Δ4GM-CSF liegt und sich von diesen nur geringfügig unterscheidet, wobei bei dem ausgewählten pH-Wert das GM-CSF und Δ4GM-CSF sehr geringe elektrische Ladungen, die gegensätzlich beladen sind, aufweist und man nur entweder GM-CSF oder Δ4GM-CSF an das Ionenausauscherharz bindet und GM-CSF wiedergewinnt.

2. Verfahren gemäß Anspruch 1, bei dem man das GM-CSF an ein Anionenaustauscherharz bindet, eluiert und anschließend einer Gelfiltrationssäulenchromatographie unterwirft.

3. Verfahren gemäß irgendeinem der vorstehenden Ansprüche, bei dem man den pH-Wert der rohen Proteinmischung auf pH5 einstellt.

## Revendications

1. Procédé permettant de séparer du GM-CSF d'avec du GM-CSF-Δ4, qui comporte le fait de mettre un mélange de protéines, comprenant du GM-CSF et du GM-CSF-Δ4, en contact avec une résine échangeuse d'ions forte, et qui est **caractérisé en ce que** l'on ajuste le pH du mélange de protéines à une valeur choisie à laquelle seul l'un des GM-CSF et GM-CSF-Δ4 est lié à la résine et l'autre ne l'est pas, laquelle valeur choisie de pH se situe entre les valeurs déterminées pour les points isoélectriques de GM-CSF et de GM-CSF-Δ4 et à seulement quelques fractions d'unité de pH d'écart de ces valeurs, ce qui fait qu'à ladite valeur choisie de pH, le GM-CSF et le GM-CSF-Δ4 portent des charges électriques qui sont très faibles et de signes opposés et seul l'un des GM-CSF et GM-CSF-Δ4 se lie à la résine échangeuse d'ions, et l'on récupère le GM-CSF.

2. Procédé conforme à la revendication 1, dans lequel le GM-CSF est lié à une résine échangeuse d'anions, élué, puis soumis à une chromatographie sur colonne de filtration sur gel.

3. Procédé conforme à l'une des revendications précédentes, dans lequel on ajuste à 5 le pH du mélange de protéines brut.
